# EUROPEAN PATENT APPLICATION

(11) **EP 3 936 872 A1**
(43) Date of publication of application: **12.01.2022**
(21) Application number: 21176577.1
(22) Date of filing: 30.06.2016
(51) Int. Cl.: G01P 15/00, A61B 5/026, A61B 5/024, A61B 5/11, A61B 5/00

(54) **DETECTION OF CONCUSSION USING CRANIAL ACCELEROMETRY**

(30) Priority: 30.06.2015 US 201514788683
(62) Divisional of application: 16818839.9
(71) Applicant: Jan Medical, Inc., Mountain View, CA 94041 (US)
(72) Inventor: LOVOI, Paul A., Saratoga, 95070 (US); NEILD, Peter J., Poway, 92064 (US)
(74) Representative: SSM Sandmair

(57) **Abstract**

A system and method for detecting brain concussion includes detecting and measuring of acceleration at one or more points on a subject's head. Sensors, which can be accelerometers placed against the head, detect and measure natural motions of the patient's head due to blood flow in the brain and resultant movement of tissue in the brain. The acceleration data are then analyzed, including as to frequency of motions of the skull at the subject location in a frequency range of about 1 to 20 Hz. An observation is then made, as compared with data corresponding to non-concussion, of a change in frequency response pattern exhibited when accelerations are plotted as a function of time or frequency, to identify probable concussion if the frequency response pattern indicates concussion. Preferably the observation and comparison are made by a computer using an algorithm.

## Description

### Background of the Invention

This application claims benefit of provisional application No. 62/019,280, filed June 30, 2014. The application is also a continuation-in-part of application Serial No. 14/565,337, filed December 9, 2014, which was a continuation-in-part of application Serial No. 11/894,052, filed August 17, 2007, now U.S. Patent No. 8,905,932, which was a regular filing from provisional application No. 60/838,624, filed August 17, 2006. These applications and patent are incorporated herein by reference.

The invention concerns noninvasive detection of anomalies of the brain, and in particular the invention is concerned with detection of concussion in a patient. The equipment and the method of the invention investigate skull motion produced by pulsatile cerebral blood flow, as measured by cranial accelerometry using one or more accelerometers attached to a patient's head.

Sports impacts are only one source of concussion. 18% of concussions are pediatric, 0 - 4 years of age. Another important segment is persistent TBI that accounts for up to 15% of all TBI. Persistent TBI does not resolve within 90 days and in some cases never resolves. Separating persistent TBI from PTSD is important to direct therapy for the symptoms.

Concussion has undergone a reevaluation in recent years with only loss of conciseness being the diagnosis of a concussion, to a list of symptoms providing the diagnosis to neurophysiological measures of concussion. So older data on the number of concussions sustained in a contact sport may be under reported by an order of magnitude or more. Approximately 300,000 sports concussions are reported annually but most authorities believe this is vastly under reported due to lack of awareness, misunderstanding of the definition of concussion and more recent realization that even what was considered minor head impacts previously are in fact concussions. Football in particular has received much media attention because of the frequency of concussions and other forms of traumatic brain injury (TBI).

A concussion is "a complex pathophysiologic process affecting the brain, induced by traumatic biomechanical forces." It is caused either by a direct blow to the head or by a blow to the body with forces transmitted to the head. A concussion results in neurological dysfunction that is usually transient and resolves spontaneously. The majority of concussions do not result in loss of consciousness; however, they result in clinical and cognitive symptoms that commonly resolve in a sequential course. Importantly, no abnormalities are seen acutely on standard neuroimaging (computed tomography and magnetic resonance imaging (MRI)). Standard neuroimaging may be useful to evaluate for skull fractures, extra-axial bleeding, and contusions; however, these are unlikely to be present after concussions, given the likely microstructural nature of this injury. Functional magnetic resonance imaging (fMRI), which measures changes in blood flow during brain activation, shows potential in the evaluation of concussion. However, fMRI is expensive, time-consuming, not applicable when metal such as orthodontic braces are present and is not easily transportable or widely available. Multimodal MRI studies, notably including diffusion tensor imaging to detect axonal pathology and susceptibility weighted imaging to detect micro-hemorrhage, have been proposed as relevant imaging modalities. Dysfunction in cerebrovascular blood flow and auto-regulation, as demonstrated by fMRI and multimodal MRI, may one day be considered an objective definitive diagnostic measure of concussion.

Given the lack of a validated definition, or of proven physiological diagnostic testing or imaging criteria, concussions in clinical practice are not diagnosed or followed to resolution by objective findings or imaging, but rather by the constellation of neurocognitive signs and symptoms that commonly accompany mild TBI. The list of these signs and symptoms is long, and many of them are included in the symptom score of Sport Concussion Assessment Tool 2 (SCAT2), and now SCAT3. Other tests used to identify and follow concussions include Standardized Assessment of Concussions, Balance Error Scoring System, and Immediate Post-Concussion Assessment and Cognitive Testing.

Because less-than-optimal recovery or repeated concussion potentially leads to worse clinical outcome, a technology to objectively detect concussion is needed. In TBI, cerebral autoregulation can be impaired and persons can develop brain edema. This perhaps affects other physical properties of brain tissue and adjacent structures. A new physiological phenomenon developed pursuant to the invention uses highly sensitive accelerometers to detect movement of the human skull during a normal cardiac cycle. Because pulsatility of cerebral blood flow is transmitted through tissue to the skull surface, we sought to measure this phenomenon using highly sensitive cranial accelerometers in resting subjects suspected of having suffered a concussion, and therefore perhaps by measuring cerebral autoregulation dysfunction or subtle amounts of brain edema.

### Summary of the Invention

With the method and system of the invention, brain concussion is detected in a human patient by detecting and measuring natural motion of the patient's head due to blood flow in the brain and resultant movement of tissue in the brain.

At least one accelerometer (and preferably at least two) is attached to the patient's head such that the accelerometer measures the acceleration of the head where attached. Acceleration of the skull is detected and measured using the accelerometer, and these measurements are sent to a computer. The data include frequency and intensity of vibrations of the skull at the location of the accelerometer, preferably in a frequency range of about 1 to 20Hz.

The data from the accelerometer are analyzed and investigated. If the data show an increase in frequency content of skull motion in selected frequency ranges above about the fourth harmonic of the patient's heartbeat, as compared with data corresponding to non-concussion, this is taken to indicate probable concussion.

The acceleration of the skull can be measured with apparatus other than actual accelerometers. Laser-based interferometers, including fiber-based interferometers can be used to measure movement at selected parts on the skull, and from these measurements acceleration intensity and frequency can be determined.

Importantly, the system and method of the invention do not require baseline data for the particular patient being tested. A graph of the data, either in the time domain or in the frequency domain, will reveal, in a patient having concussion, a particular pattern that will not be seen in a graph of data from a non-concussed patient. The pattern indicating concussion can be observed visually by a trained physician or technician, or the data can be analyzed using an algorithm operated by a computer.

The algorithm can be in several forms, but primarily it will detect, as noted above, an increase in energy of skull motion in frequency ranges above about the fourth harmonic of the patient's heartbeat. In a mathematical algorithm these data need to be "normalized", and in one form of algorithm the averaged energy of, for example, the fifth and sixth harmonics of the heartbeat is compared against (i.e. divided by) a maximum value of one or more or an average of several of the lower harmonics, below the fourth harmonic. This ratio, which is often called herein R₁, is compared against a selected threshold. Such a threshold, in one embodiment of the invention, is set at 1.0, but different thresholds can be selected, based on desired levels of sensitivity and specificity. The higher the threshold for R₁ is set, the lower the sensitivity but the higher the specificity, and vice versa.

The waveform is obtained by averaging data over a number of heartbeats, which can be, for example, about 45 heartbeats, or a range of about 40 to 60 heartbeats.

Preferably another ratio factor is also included in the described algorithm, which can be called R₂. R₂ represents "normalized" data from the eighth and ninth harmonic peaks. The average value of those peaks is also divided by a maximum or average of one or more of the lower harmonics, the same denominator used for R₁. In one embodiment of the algorithm the R₂ threshold is set at 0.66. Thus, concussion is indicated if R₁ ≥ 1.0 and R₂ ≥ 0.66. Concussion is contra-indicated in the case where both R₁ and R₂ are below the set thresholds.

An important aspect of the invention is the ability to detect concussion early. Typically peak data exceeding thresholds, in the procedure described above, are not exhibited for a few days after a concussive event, e.g. about day 4. The factors R₁ and R₂ may not cross the thresholds until day 3 or day 4, or even longer in some cases. It is known through cognitive concussion testing that some delay occurs in concussion manifesting itself (although maximum indications in cognitive testing tend to occur earlier than those from the subject algorithm). However, the system of the invention can detect the rise of these factors toward concussion even in the first day or two after an event of trauma to the head. This is accomplished by observing the velocity of change in the patient's R₁ and R₂, values in the period leading up to the concussion symptoms exhibiting themselves, referred to herein as the period of "developing" concussion, typically about four days. With the system of the invention, the data can be observed as showing clear and definitive movement toward crossing the thresholds for R₁ and R₂, in a patient with concussion. Thus, days before the data analysis shows positive for concussion, the Velocity of movement in that direction will indicate concussion. There previously existed no other reliable means for detecting concussion in this "developing" period.

After R₁ and/or R₂ reach peak values, i.e. in the case of R₁ the maximization of the frequency-domain peaks at about the fifth and sixth harmonics of the patient's heartbeat, a period of usually several weeks of declining values ensues, called the "recovery" period.

Although the data comparison step to determine concussion can be accomplished by a trained physician reviewing a graph, it is preferably performed by an algorithm operated on a computer, which can be the same computer to which the raw data is fed.

Importantly, the data corresponding to non-concussion, to which the accelerometer-derived data is compared, preferably are data observed from a plurality of normal persons without concussion; baseline data for the particular patient being examined is not required. In contrast, with Cognitive testing pre-season baseline data for football players, for example, is required.

Another important aspect of the invention is the use of a Campbell diagram as an indicator of concussion, or as a confirmation. The responses of a concussion patient's brain to vascular pulsing are frequency dependent, and the Campbell diagram was developed for frequency dependent functions, such as turbine design in jet engines. Vibration response in a turbine tends to be different at different revolution speeds. Since concussed brain responses are also frequency dependent, i.e. heartbeat rate dependent, a typical waterfall diagram of the gathered data on a patient will usually not produce sharp lines - a patient's heartbeat rate can vary with time. However, the data can be plotted to produce sharper lines, as eigenfrequency lines, if heart rate is represented on the vertical axis and frequency on the horizontal axis. The eigenfrequency of a concussion patient typically are essentially radial lines emanating and fanning out from the theoretical point 0,0. In particular if the "hot" color bands (red, orange, indicating high intensity) follow such radial, fanning lines, this indicates the harmonics of the system are changing in frequency with the driving function. That is to say, as the speed decreases, then the harmonics as an ensemble decrease in a pattern of the fan going down to zero. If, on the other hand, the structure is not responding to the driving force, i.e. to the frequency, but is simply a structural response, then the bands will be vertical. Therefore, one can use these bands to detect whether this is a structural change. It appears that in the normal brain (without concussion), the bands tend to be vertical; changing the heart rate does not shift the peaks of the harmonics. With concussion, changing the heart rate does change the position of the harmonics such that they follow the eigenfrequency lines down to zero. This is another method of detecting concussion and potentially detecting it much earlier than the R₁ or R₂ or velocity can do. The Campbell diagram provides an efficient reference that can be used as a primary determination for concussion indication (or not), or which can be used as a check against the conclusion reached via another algorithm such as that discussed above. The harmonic peak locations can be compared to the eigenfrequency lines by a correlation function to determine how well the structure responds to the varying heart rate.
The method of the invention also encompasses additional techniques to optimize the identification of peaks on the plot of accelerometer data in the frequency domain, using algorithms. As discussed above, one preferred algorithm developed pursuant to the invention has been to look at the values at the fifth and sixth harmonics as a basis for R₁, and also at the eighth and ninth harmonics as a basis for R₂. When an algorithm is applied based on the harmonics, the peaks will seldom fall precisely at the harmonic frequencies being investigated. In spite of this, the algorithm functions very well, with high sensitivity and specificity. Improvement can be made, however, by more closely identifying the actual frequency locations of the peaks that tend to occur around the fifth and sixth harmonics and also around the eighth and ninth harmonics. This can be done visually on a computer monitor, and it could also be done using a pattern recognition program, similar to those used for facial pattern recognition. Techniques are also available to identify these peaks accurately using an algorithm that does not use the visual appearance of a time domain or frequency domain chart. As one example, the algorithm can include a maximizing or optimizing feature by which, after the data are analyzed at the fifth and sixth harmonics and the eighth and ninth harmonics (as well as at lower harmonics as discussed above), the frequency under examination can be shifted up or down for each of the fifth, sixth, eighth and ninth harmonics to find the peak value within a selected frequency band of the nominal harmonic. By this method the actual peaks of the four approximate harmonics that are used to calculate R₁ and R₂, can be pinpointed, for more accurate analysis.

By the system and method of the invention, concussion can be determined with a high degree of sensitivity and also specificity, without any baseline data for a patient, and it can be determined at a very early stage, by observing results at repeated intervals (daily or more frequently) immediately following a potential concussive event. In addition, the period of recovery from concussion can be monitored accurately, and the point of and the time of actual recovery can be confirmed. These and other objects, advantages and features of the invention will be apparent from the following description of a preferred embodiment, considered along with the accompanying drawings.

### Description of the Drawings

Figures 1A and 1B are frontal and side views schematically indicating accelerometer sensors located on a patient's head according to the invention.
Figure 2A is a block diagram showing components of the system and method of the invention.
Figure 2B is a flow chart showing an embodiment of the process of the invention.

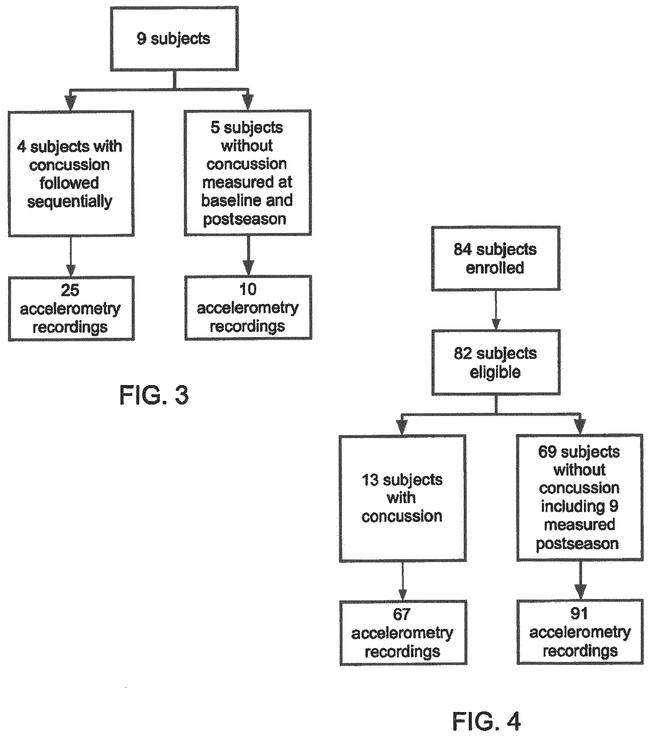

Figure 3 is a chart indicating Phase 1 testing of subjects using the system of the invention.
Figure 4 is a chart indicating Phase 2 testing of subjects.
Figure 5 is a frequency plot of a particular subject, showing a pattern indicating non-concussion.
Figures 6A to 6D are FFT plots of four different subjects, all indicating concussion.
Figure 7 is a chart of the R₁ factor of the algorithm of the invention against time.
Figures 8A - 8C are charts plotting R₁ and R₂ factors for several different populations of recordings using the system of the invention.
Figure 9 shows an example of a Campbell diagram, as an indication of concussion.
Figures 10 and 11 show examples of time-domain waveforms for typical non-concussed and concussed subjects.

### Description of Preferred Embodiments

Preferred embodiments of the invention are explained below in terms of system components and tests that have been performed. Initial discussion is in regard to testing performed to identify a specific pattern indication of concussion using cranial accelerometry (phase 1), to test the identified pattern against blinded data to verify its efficacy in detecting concussion (phase 2).

In all testing, subjects had accelerometry measurements and concurrent two-lead electrocardiograms. In players with a concussion, multiple sequential measurements were obtained. Sport Concussion Assessment Tool 2 was used to assist clinical determination of concussion.

As explained in greater detail below, phase 1 was the process whereby accelerometry data indicative of a concussion pattern were determined, and phase 2 was evaluation of these findings against a blinded set of accelerometry data.

The following explanation pertains to methods used to acquire data for both phases 1 and 2.

### Accelerometry Measurements/Equipment and Methodology

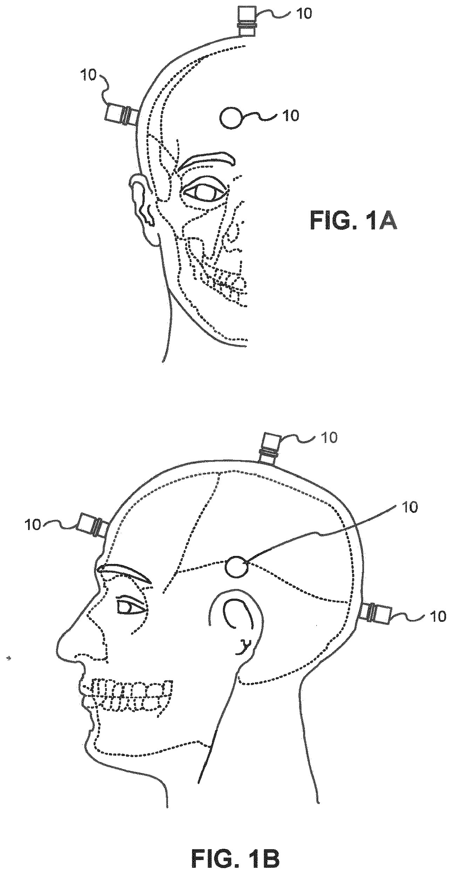

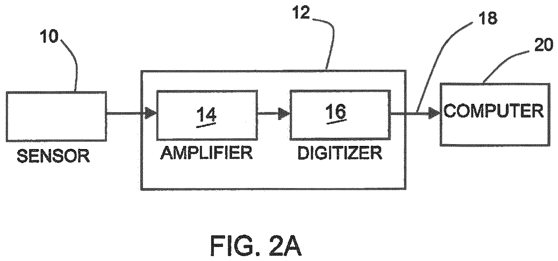

This investigational device comprises a headset with accelerometer 10, as indicated in Figures 1A and 1B, as well as a data Collector unit 12 with amplifier 14 and digitizer 16, a detachable coaxial cable 18, and laptop computer 20, all shown schematically in Figure 2A. The system collects and stores data derived by sensing small motion produced by pulsatile cerebral blood flow and its impact on the skull. This is accomplished by six highly sensitive accelerometers 10 that are positioned within an adjustable headset (not shown) to contact the scalp bitemporally, bifrontally, occipitally, and at the skull vertex. Transduced accelerometer data are digitized with the digital signal processor 12 and sampled with the laptop computer 20. An omnidirectional sound pressure level (SPL) sensor (not shown) is positioned on the top of the headset for the purpose of ambient noise eradication. Correlated noise patterns between the SPL sensor and accelerometer data may be removed during processing. A heart rate sensor (not shown) is also provided using a PPG (photoplethysmograph). Note that concussion can be detected using only a single temporally-placed sensor, although two opposed temporal sensors are preferred.

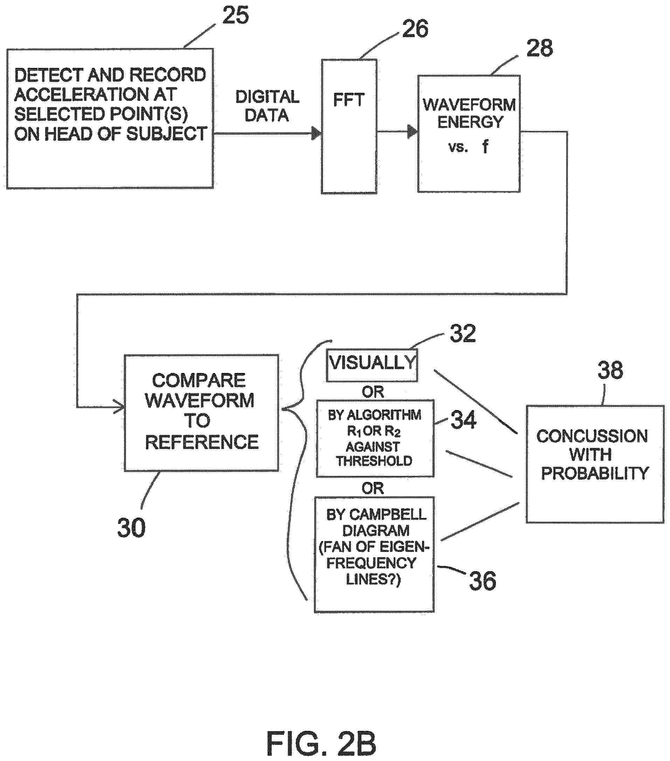

Figure 2B is a simplified flow chart outlining the method of the invention. The block 25 indicates detection and recording of acceleration at one or more selected points on the head of a subject. As noted above, this can be done using accelerometer sensors 10 placed against the head, but other methods and equipment for detecting acceleration could also be used. For example, a laser interferometer could be used to monitor extremely small motions of the skull, the second derivative of position being acceleration. In any event, this data is digitized and, in a preferred embodiment, a fast Fourier transform (FFT) is performed on the data, as indicated at 26. A waveform is produced, per the block 28, as a plot of energy or intensity versus frequency. This waveform is compared to a reference waveform (block 30), to determine whether concussion is indicated. The dependent blocks 32, 34 and 36 indicate that this comparison can be done visually, by algorithm, or, as explained below, via a Campbell diagram. If comparison is done visually, the FFT waveform will be shown on a computer monitor and can be compared to a typical non-concussion waveform or to a typical concussion waveform, or both. A mathematical algorithm developed according to the invention is explained below, as is the Campbell diagram. The block 38 indicates the system provides a conclusion as to whether concussion is indicated, and the probability of accuracy can accompany this conclusion.

### Sport Concussion Assessment Tool

Sport Concussion Assessment Tool 2 (SCAT2) was documented and accompanied all accelerometry recordings except those obtained postseason. Sport Concussion Assessment Tool 2 is a standardized tool introduced at the 2008 third Consensus Conference on Concussion in Sport held in Zurich. All scores from sections of the SCAT2 tool contribute to a possible total of 100 points. Scoring fewer points in any section lowers the aggregate score. For instance, baseline scores for high school athletes averaged 88.3 ± 6.8. In our study, declines in SCAT2 scores were used to assist in the clinical determination of whether a head impact resulted in a concussion. SCAT2 or SCAT3 or similar neurocognitive testing can be used for other indications of concussion such as accidents.

### Subjects and Sample Size

Candidates for this observational study included all football players (grades 9-12) at a northern California high school during the 2011 football season.

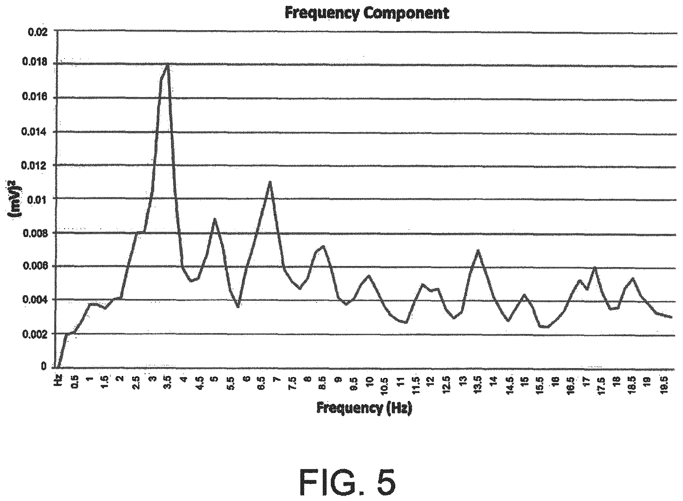

Visual inspection of data from concussed and non-concussed subjects revealed that in the non-concussed subjects, cranial motion had a series of peaks that typically declined (diminished in amplitude) with increased frequency. Figure 5 shows the frequency components plot obtained from the cranial accelerometry recording of a typical non-concussed subject. It is notable that the fifth and sixth harmonic peaks have lower amplitude than do the first to third harmonic peaks. This was a consistent finding in subjects without concussion.

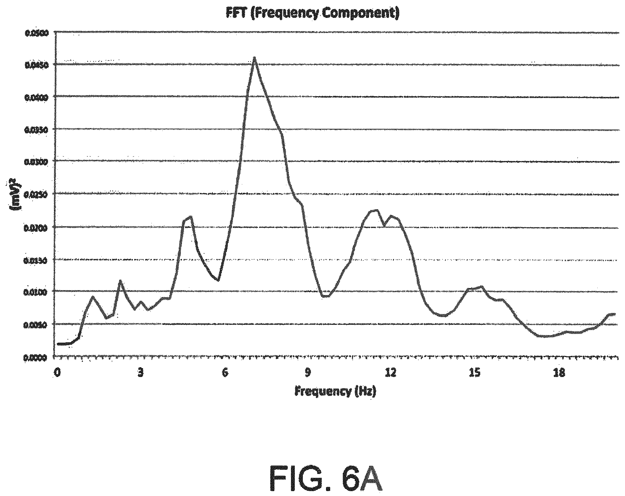

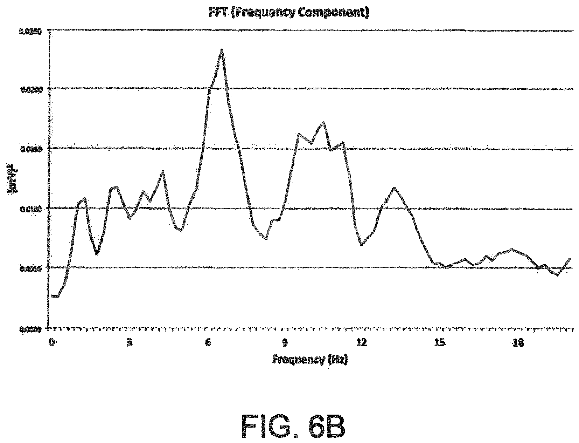

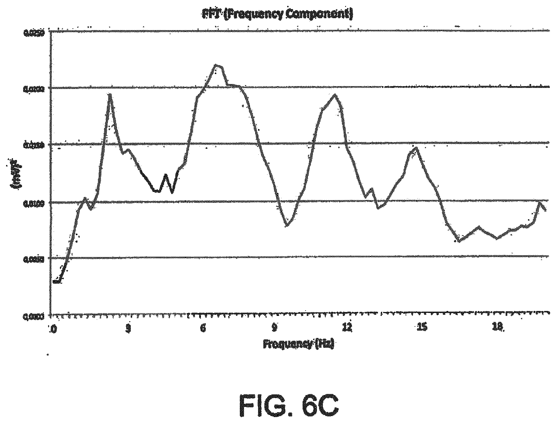

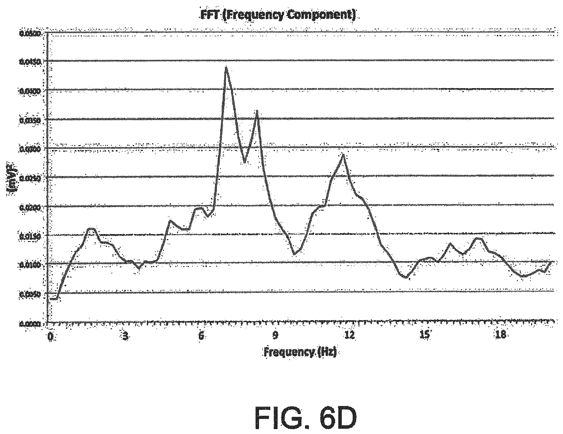

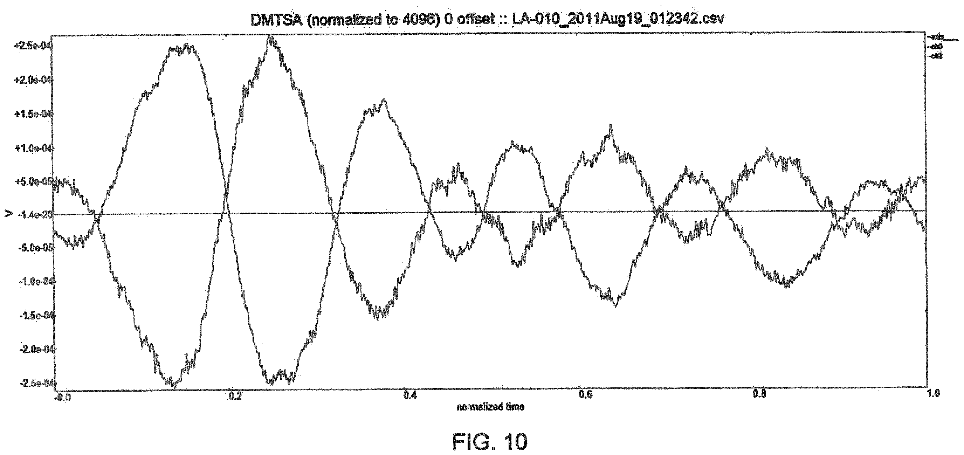

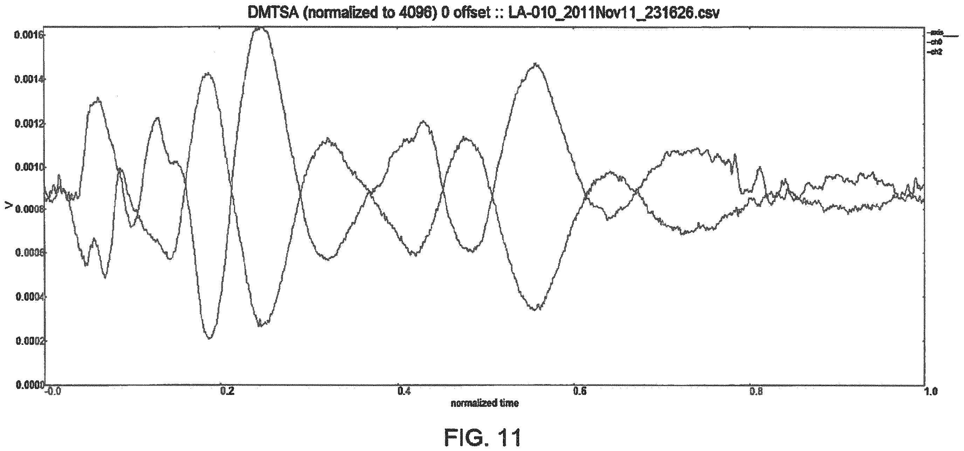

In the subjects with concussion, several additional peaks, representing higher frequencies, appeared. These new peaks arose at frequencies including and between 5 and 12 times the heart rate frequency. Three high-frequency ranges were used in algorithm development to differentiate between concussed and non-concussed subjects. Figures 6A - 6D show the frequency component plots of four concussed subjects. Note that the fifth and sixth harmonic peaks have higher amplitude than do the first to third harmonic peaks. This increase in higher frequency harmonic content continues in paired bands at about 5 to 6 times the heart rate, 8 to 9 times the heart rate and 11 to 12 times the heart rate. This increase in higher frequency harmonic content can also be observed in the time-domain waveforms as shown in Figures 10 and 11, described below.

Several algorithms are described to quantify the differences between concussed and non-concussed subjects. Other algorithms are also possible. One such algorithm defines up to three factors (R₁, R₂ and R₃) based on the relative height of the peaks, with the higher frequencies compared with the lower. The lower three harmonic values (first, second and third) define the denominator for these three variables. R₁ is defined as the average of harmonics 5 and 6 amplitude divided by the maximum of the lower three harmonic amplitudes. R₂ is the average of harmonics 8 and 9 amplitude divided by the maximum of the lower three harmonic amplitudes. R₃ is the average of harmonics 11 and 12 amplitude divided by the maximum of the lower three harmonic amplitudes. Higher values of R₁ through R₃ indicate higher energy within the higher frequencies of skull motion. R₁ and R₂ can be used to create a Boolean value (true or false). This Boolean value is true if R₁ ≥ 1.0 AND R₂ ≥ 0.66; a Boolean value of "true" defines concussion. R₁ and R₂ (or either of them) can also be used as a continuous variable that follows the clinical time course of concussion. Likewise R₃ can used to discriminate between concussed and non-concussed subjects by its increase over non-concussed subjects' values and by comparing it to R₁ and R₂. In this case the values of R₁, R₂ and R₃ should diminish with increasing harmonic number.

The sensitivity and specificity of the described algorithm in detecting concussion from a set of subjects playing American football are shown in Tables 1 and 2.

**TABLE 1. Sensitivity in Detecting Concussion**

| **Clinically Concussed Subjects*** | **C (True +)** | **NC (False - )** | **Sensitivity, %** | **CI, %** |
|---|---|---|---|---|
| 13 | 10 | 3 | 76.9 | 46 - 94 |

| | | | | |
|---|---|---|---|---|
| *Thirteen subjects were confirmed by SCAT2 and clinical assessment to have suffered a concussion. The concussion algorithm pattern was seen in 10 of these subjects and was not seen in 3 of these subjects. **C**, concussion; **NC**, no concussion, **CI**, confidence interval (P = 0.95) | | | | |

**TABLE 2. Specificity in in Detecting Concussion**

| | **No. Recordings** | **C (False +)** | **NC (True -)** | **Specificity, %** | **CI, %** |
|---|---|---|---|---|---|
| Baseline and postseason recordings* | 18 | 5 | 13 | 72.2 | 46 - 89 |
| Baseline recordings⁺ | 15 | 0 | 15 | 100.0 | 74 - 100 |
| Baseline recordings‡ | 58 | 7 | 51 | 87.9 | 76 - 94 |
| Total recordings | 91 | 12 | 79 | 87.0 | 78 - 93 |

| | | | | | |
|---|---|---|---|---|---|
| * Baseline and postseason recordings from subjects (n = 9) with no reported or suspected concussion for the duration of study. ⁺ Baseline recordings from subjects (n = 15) who, at some time after baseline recording, reported a possible concussion and had multiple recordings related to that event. Thirteen of these 15 subjects were confirmed to have a concussion by SCAT2 and clinical assessment (Table 1). ^{‡}Baseline recordings from subjects with no reported or suspected concussion at the time of recording. **C**, concussion; **NC**, no concussion, **CI**, confidence interval (*P* = 0.95) | | | | | |

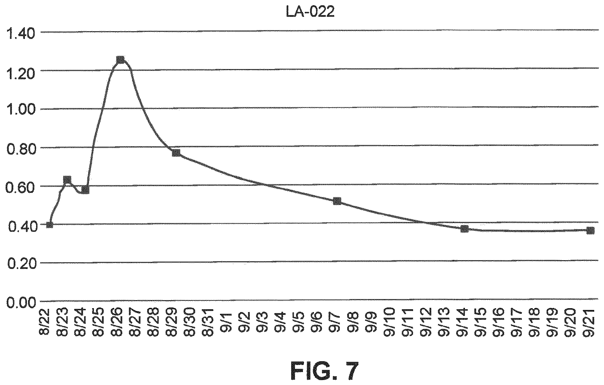

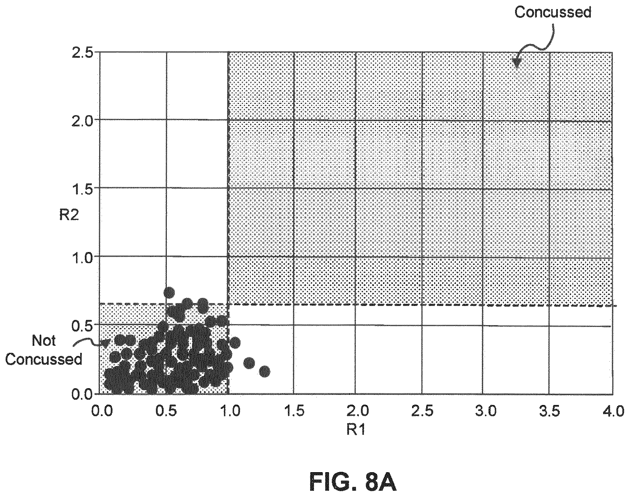

To gather insight into the time course of the presence of the concussion pattern, we plotted factor R₁ over time in subjects who were able to provide multiple recordings after concussion. Figure 7 is an example of R₁ plotted against time for one subject with a concussion. The first cranial accelerometry recording was the day following the suspected concussive event (SCB). R₁ generally varied smoothly and followed the clinical course of concussion. However, in subjects with multiple recordings, the concussion pattern returned to a normal pattern at a time beyond self-reporting of symptoms as measured by neurocognitive testing (catalog and assessment of symptoms). This is notable in that concussion patients tend to be cleared by neurocognitive to return to sports activities too early. Analysis indicates that the time course of this pathophysiological change begins immediately after concussion and outlasts the duration of clinical concussion before it eventually resolves. Using the method described above to detect concussion leads to delays of 0 to 7 days from injury to detection. Another factor, R₃, the rate of change of R₁ or R₂ with time (R₁ or R₂ velocity) may be used to predict a concussion within hours of an injury. A given individual, when measured repeatedly, has a cluster of R₁ and R₂ which may be similar to that as shown in Figure 8A, at lower left. When a recording provides an R₁ and R₂ that differs by an amount greater than the average variation in typical subjects, then that change provides a measure of concussion detection. The more recordings and factors obtained, the more confident the diagnosis of concussion. These factors could be obtained from continuous recordings to further improve the detection speed.

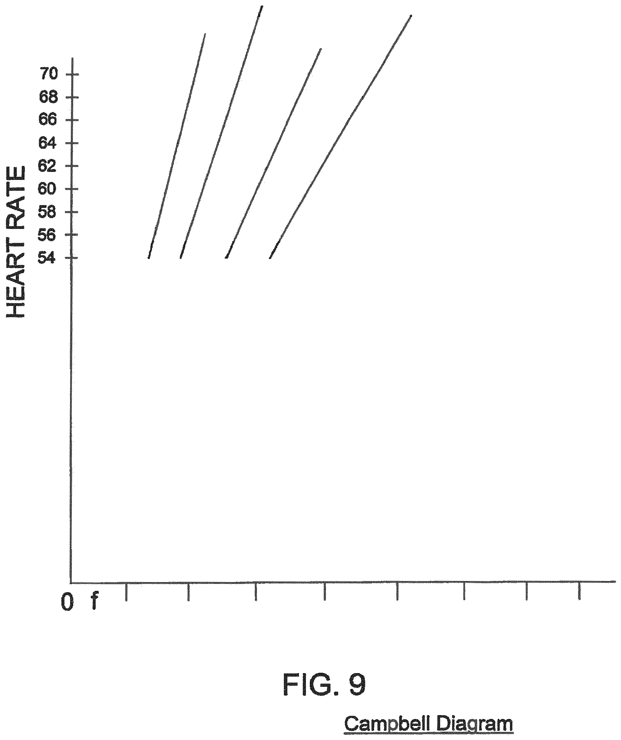

Likewise a visual examination of the time domain or frequency domain plots of the average brain pulse or a visual examination of both domains of the average brain pulse provides a rapid method of detecting concussion, as explained below with reference to Figures 10 and 11. These changes can be further displayed in another visual algorithm using color intensity waterfall (CIWF) plots. In this type of plot, the overlap FFT of the subject recordings are plotted in a horizontal line with frequency on the horizontal line axis. The intensity of each frequency bin of the FFT is color-coded; a typical color-coding uses blue for the lowest value and red as the highest value following the color spectrum for values in between. Each subsequent recording FFT is plotted below the previous recording so that the vertical axis is time. The convention is that time increases toward the plot origin. With this type of plot, shifting harmonic content is observed as increasing color intensity over time in the 5 to 13 harmonic frequencies. Another related algorithm to detect concussion is a variation of CIWF, the Campbell diagram. The Campbell diagram was developed to detect damaging harmonic resonances in rotating machinery such as jet turbine engines. In human subjects the heart rate varies with respiration, providing a natural variation to the brain pulse, so that the Campbell diagram is based on heart rate. Each of the individual heart rate brain pulse recordings is sorted. The CIWF plot is modified so that the vertical axis is no longer time but heart rate with the highest heart rate FFT line plots farther from the plot origin and the lowest heart rate FFT line plots closer to the plot origin; see Figure 9. Lines, called eigenfrequency lines, are drawn from each harmonic on the highest frequency FFT line plot, with each eigenfrequency line connecting to the waterfall plot origin. If the changes in the brain pulse are due to a concussion, the subsequently lower heart rate frequency FFT line plots will have lower harmonic peak locations, aligning with each corresponding eigenfrequency line, and higher heart rate FFT plots will have higher harmonic peak locations. Correlation of the harmonic peak location to the eigenfrequency lines, fanning out essentially from the plot's origin, provides a factor for concussion detection. This fanning out Campbell diagram seems not to occur without concussion.

The precise pathophysiology of concussion remains undefined, but likely is related in some way to injury that includes blood flow abnormalities, fluid accumulation, and/or structural changes. A shift of harmonic energy toward higher frequencies (or an increase at the higher frequencies) is likely caused by an increase in brain resonance from the force induced by pulsatile blood flow entering the skull. This may be caused by a stiffer brain and could be accounted for by brain edema or perhaps changes in cellular structure. However, brain edema has not been demonstrated in persons with clinical concussion, so this explanation is by no means certain. It is possible that disruption of cerebral autoregulation might produce phase changes in brain resonance and result in higher harmonics of the cardiac-induced forcing function into the closed skull space.

It was not part of our study to determine which subjects continued to suffer from concussion at the end of the season as determined by SCAT2 test results. We did not obtain SCAT2 test results from subjects at the end of the season, so cannot conclude who did or did not continue to register a concussion by that test method. Throughout the study, we used clinical judgment to determine the presence or absence of concussion. It is a recognized limitation of concussion diagnosis that clinical judgment and psychometric testing are subject to variability. However, this clinical judgment could not have been biased by knowledge of accelerometry data because these data were kept blinded until they had been fully collected and analyzed.

The observation that in certain subjects the concussion pattern returned to the non-concussion pattern after reporting of symptoms resolution but then returned to the concussion pattern after exercising suggests that return to activity is perhaps premature if it is not based on an objective physiological determination.

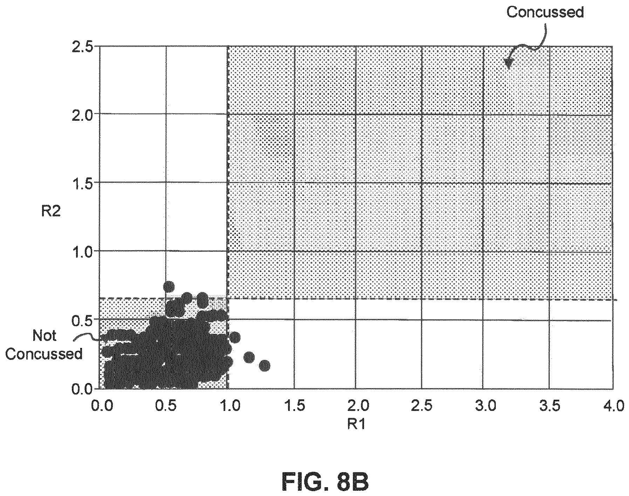

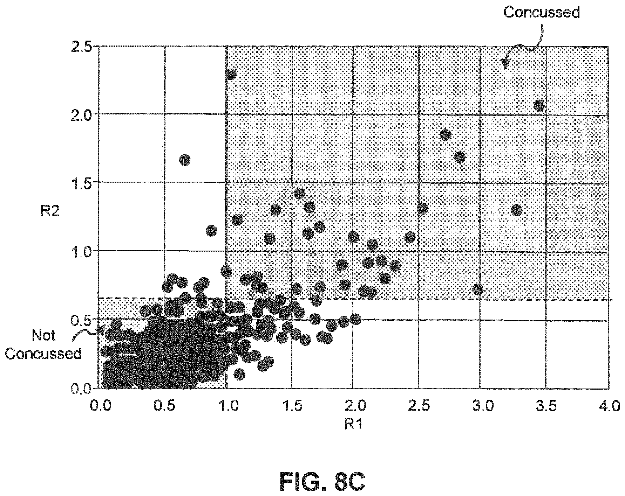

Figures 8A, 8B and 8C are population type charts showing results of tests on a number of subjects, plotted on a graph of R₁ and R₂, with dotted lines indicating thresholds of R₁ (vertical dotted line) at 1.0 and a threshold for R₂ (horizontal dotted line) at 0.66, according to one preferred embodiment of an algorithm. When both R₂ < 1.0 and R₂ < 0.66, the conclusion is non-concussion (lower left of chart). Also, if either the R₁ or R₂ is below threshold, the conclusion is non-concussion (a few in Figure 8B have only one threshold met). If both these factors equal or exceed the threshold, i.e. the R₁, R₂ plot is in the upper right of the chart, concussion is indicated according to this algorithm. All three charts indicate specificity of the algorithm of the invention.

In Figure 8A twenty-two subjects were evaluated. These were athletes in non-contact sports, aged 14 to 18. The recording sequence was two recordings every week for four weeks, for a total of 176 recordings. Of these, 159 resulted in valid recordings (due to the inability of the PPG sensor to function properly in bright sunlight). As shown on Figure 8A, all tests come squarely in the lower left non-concussion region of the chart. Since there were zero false positives, the specificity of the test and algorithm was 100%, as to each of the 22 subjects and as to each of the 159 valid recordings. Since all subjects reported no possibly concussive events in recent months before testing or during the test, it appears probable that none of these tests represent a false negative.

Figure 8B is another plot of R₁ and R₂ for 1205 recordings taken over approximately two years, from eight subjects, all of whom had no reason to believe any concussion event had occurred. No recording indicated concussion, for a specificity of 100%. As the chart shows, a few recordings were outside the lower left quadrant, with R₁ ≥ 1.0 or R₂ ≥ 0.66, but none exceeded both thresholds. Those are considered not concussed.

Figure 8C is a similar chart but for 1784 recordings, including the 1205 reported in Figure 8B. The tested population included persons known to have dementia, pediatric DKA (diabetic ketone acidosis), stroke, vasospasm and 91 impact sports subjects who could possibly have had concussions, but without diagnosis. As the chart of Figure 8C shows, 36 of the total population of 1784 recordings indicated concussion, with both R₁ and R₂ exceeding thresholds. Since none of the persons tested was known to have concussion, all must be assumed as non-concussed. Still, this produces a specificity of 98%, which is very high.

Note that many of the subjects reported in the testing and chart of Figure 8C had other structural brain issues, including dementia, stroke, ischemic stroke, DKA and vasospasm. 42 had dementia and 86 had DKA, and none of these were recorded as concussed. 25 had stroke and 296 had vasospasm; only one stroke patient was recorded as concussed, and only 13 vasospasm subjects were shown as concussed. 91 of the subjects were involved in impact sports, and 12 were shown as having concussion. It is possible (but unknown) that these were true positives, but for purposes of this specificity analysis they are assumed as false positives.

Figures 10 and 11 show plots of accelerometer readings taken on a subject with only two opposing accelerometer sensors, at left and right temporal lobes. These are plots of acceleration events during a single heartbeat (at a heartbeat rate of 1Hz), against time (i.e. in the time domain). Figure 10 is from a subject without concussion; Figure 11 is a plot from the same subject but after a concussion event. Figure 10 indicates non-concussion, while Figure 11 indicates concussion. The opposed positive and negative values on the charts represent data from the two opposed temporal accelerometers. Both charts have data averaged together over 45 heartbeats. From Figure 10 it is seen that during the systole portion of a heartbeat, which is up to about 0.3 on the time scale, only two large positive peaks appear (one from the left sensor and one from the right sensor). However, the graph of Figure 11 is strikingly different, with four prominent positive peaks showing during the systole portion of the heartbeat. This is a typical differentiating pattern to indicate non-concussion or concussion in a graph of acceleration in the time domain. Other differences occur in the remainder of the heartbeat, but the systole portion is the most prominent. As an alternative, analysis can be done by counting zero crossings with up to 8 or 10 being typical of non-concussed and a higher number being typical of concussed.

The described methodology identifies and utilizes a novel, unique pattern of cranial accelerometry that correlates with human concussion. It is influenced by movements of the brain within the skull that occur during cardiac-induced blood flow pulsations.

The terms "intensity of frequency", "frequency content", and "amplitude of signal data" are used in the above description and in the claims. These terms refer to how much of each of a series of particular frequencies occurs in a waveform analyzed by the algorithm, through a band of frequencies (e.g. 1 - 28 Hz). In the frequency domain they refer to the amplitude, i.e. height of signal data in the plot of amplitude (which can be called energy or power) versus frequency.

The above described preferred embodiments are intended to illustrate the principles of the invention, but not to limit its scope. Other embodiments and variations to these preferred embodiments will be apparent to those skilled in the art and may be made without departing from the spirit and scope of the invention as defined in the following claims.

### The invention encompasses the following examples A to J

A. A method for detecting brain concussion in a human patient, by detection and measuring of natural motions of the patient's head due to blood flow in the brain and resultant movement of tissue in the brain, comprising:
   detecting and measuring motions of the skull at one or more selected points on the skull using a sensor, connected to a computer, including recording frequency of motions of the skull at the location of the sensor, in a frequency range of about 1 to 20 Hz, and
   observing, as compared with data corresponding to non-concussion, an increase in frequency content of skull motion in frequency ranges above about the fourth harmonic of the patient's heartbeat, thus to indicate probable concussion.
B. The method of example A, wherein the data corresponding to non-concussion is data from human subjects other than the patient.
C. The method of example A, further including, if probable concussion is indicated, monitoring progression of recovery from concussion by detecting a progressive decrease over time in frequency intensity of skull motion in said frequency ranges.
D. The method of example A, wherein the observing step is performed using an algorithm operated by the computer.
E. The method of example D, wherein the algorithm includes a calculation of a ratio between a first value of amplitude of signal data representing skull motions at frequencies which are approximately at the fifth and sixth harmonics of the patient's heartbeat, and a second value of amplitude of signal data comprising one or more or an average or two or more of the lowest three harmonics of the patient's heartbeat, said ratio defining a factor R₁, with concussion or non-concussion indicated by whether R₁ exceeds a preselected threshold value.
F. The method of example E, wherein the R₁ threshold value is about 1.0.
G. The method of example E, wherein the second value of amplitude of the R₁ factor is a maximum value of the first three harmonics of the patient's heartbeat.
H. The method of example E, wherein the detector used in the step of detecting and measuring motions of the skull comprises attaching at least one accelerometer to the patient's head such that the accelerometer follows the motions of the head at the point of the accelerometer's attachment to the head.
I. The method of example E, wherein the algorithm further includes a calculation of a factor R₂, as a ratio of the average value at the eighth and ninth harmonics of the patient's heartbeat to one or more or an average of a plurality of the values of the first three harmonics of the patient's heartbeat, and wherein the factor R₂ is compared to a preselected R₂ threshold as a second indicator of concussion or non-concussion.
J. A method for detecting brain concussion in a human patient, by detection and measuring of natural motions of the patient's head due to blood flow in the brain and resultant movement of tissue in the brain, comprising:
   attaching at least one accelerometer to the patient's head such that the accelerometer follows the motions of the head at the point of the accelerometer's attachment to the head,
   detecting and measuring motions of the skull with the accelerometer, connected to a computer, including recording frequency of motions of the skull at the location of the accelerometer, in a frequency range of about 1 to 20 Hz,
      using an algorithm operated by the computer, observing an increase in frequency content of skull motion in frequency ranges between about the fourth harmonic and the seventh harmonic of the patient's heartbeat, and calculating a ratio of frequency content within said frequency ranges divided by a maximum or average of frequency content within harmonics below the fourth harmonic, and determining whether such ratio exceeds a predetermined threshold, thus to indicate probable concussion.

## Claims

1. A method for detecting concussion in a patient, comprising:
using a device capable of measuring acceleration of a surface or object, noninvasively measuring acceleration of at least one point on the head of the patient, such accelerations being from pulsatile blood flow in the cranium,
observing frequencies of such accelerations,
selecting from said frequencies a band of higher frequency below 20 hertz,
selecting a band of lower frequency below 20 hertz, and observing a shift in balance between energy in the selected higher frequency band and energy in the selected lower frequency band, as compared to a reference balance, and diagnosing concussion based on said shift in balance.

2. A method for detecting brain concussion in a human patient, by detection and measuring of natural motions of the patient's head due to blood flow in the brain and resultant movement of tissue in the brain, comprising:
detecting and measuring motions of the skull at one or more selected points on the skull using a sensor, connected to a computer, including recording frequency of accelerating motions of the skull at the location of the sensor, in a frequency range of about 1 to 20 Hz, and
observing, as compared with data corresponding to non-concussion, a change in frequency response pattern exhibited when accelerations are plotted as a function of time or frequency, and
identifying probable concussion if the frequency response pattern is concussion indicative.

3. The method of claim 1, wherein the frequency response pattern is observed in the frequency domain.

4. The method of claim 1, further including, if probable concussion is indicated, monitoring progression of recovery from concussion by detecting a progressive decrease over time in frequency intensity of skull motion in said frequency range.

5. The method of claim 1, wherein the observing step is performed by a computer via an algorithm analyzing the frequency response.
